# EUROPEAN PATENT SPECIFICATION

(11) **EP 3 731 883 B1**
(45) Date of publication and mention of the grant of the patent: **25.11.2026**
(21) Application number: 18826400.6
(22) Date of filing: 26.12.2018
(51) Int. Cl.: A61L 9/12

(54) **SUPPORT FOR VOLATILE SUBSTANCE-DIFFUSING WICKS**
TRÄGER FÜR EINE FLÜCHTIGE SUBSTANZ DIFFUNDIERENDE DOCHTE
SUPPORT POUR MÈCHES DE DIFFUSION DE SUBSTANCE VOLATILE

(30) Priority: 27.12.2017 ES 201731475
(43) Date of publication of application: 04.11.2020
(73) Proprietor: Zobele Holding SpA, 38100 Trento (IT)
(72) Inventor: CAMARERO DÍEZ, Roberto, 08019 BARCELONA (ES); MASÓ SABATÉ, Jordi, 08019 BARCELONA (ES)
(74) Representative: Herrero & Asociados, S.L.
(86) International application number: PCT/EP2018/086884
(87) International publication number: WO 2019/129789

(56) References cited:
- EP-A1- 1 103 479
- EP-A2- 1 779 871
- WO-A1-2006/004891
- WO-A1-2006/061803
- WO-A2-2008/036263
- US-A1- 2003 132 308

## Description

The present invention relates to a support for volatile substance-diffusing wicks, particularly a support which is placed in the neck of a liquid-filled container.

### Background of the Invention

Volatile substance diffusers formed by a container, for example, a bottle, filled with a liquid containing volatile substances, are commonly used. For the diffusion of these volatile substances, a wick which is impregnated with liquid at one of its ends and exposed to the open air at the other end, diffusing volatile substances, is used.

In order to place the wick in a substantially vertical position so that it can diffuse the volatile substances properly, a certain type of support comprising a housing for placing the wick is often used.

The drawback of these supports used today is that they do not prevent the wick from being voluntarily removed for reuse or the liquid from coming out accidentally, for example, in the event of the container tipping over. This is particularly important if the liquid spills over as it usually contains aggressive substances, such as solvents, which can damage the surface on which the container is placed.

WO2006061803A1 discloses a device, and the consumer articles associated therewith, for dispensing an active composition into the surrounding space. The device comprises an active liquid, a reservoir holding the active liquid, a wick/emanator superstructure composed of a wicking part and an emitting part, the latter having an evaporative surface to be directly exposed to the surrounding space when the device is activated and being housed in a moveable housing assembly.

EP1103479A1 discloses a plug comprising a body permanently coupled to the neck of a container for an air freshener or insect repellent liquid, with evaporation occurring through a wick axially coupled in a concentric passage of the plug itself.

WO2008036263A2 discloses a refill for a volatile material includes a container having a reservoir portion and a neck portion. The refill further includes a plug assembly disposed within the neck portion of the container and a channel through a central portion of the plug assembly.

WO2006004891A1 discloses an apparatus for dispensing a volatile substance including an electromechanical dispenser attached to a pivot arm, and a cartridge for holding a plurality of reservoirs each containing a volatile substance. The cartridge is rotatable such that any one of the reservoirs can be positioned in an engagement position, in which the volatile substance can be dispensed.

EP1779871 A2 discloses a diffusing device for volatile substances, comprising a container designed to contain a volatile liquid, a wick partially inserted in the container and partially disposed outside it, and an under-cap interposed between the neck of the container and the wick, to hold the latter in position, wherein means are provided such as to prevent the wick and or the under-cap from coming out of the container in the event of traction on the outer part of the wick.

US2003132308 A1 discloses a container for volatile substances which can be evaporated by an evaporation device is disclosed having a container body in which the volatile substances are contained. A container neck extends away from the container body in which a container opening is formed. Furthermore, a wick retaining insert in the form of a cylindrical sleeve is provided that can be placed in the container opening of the container neck.

Therefore, an objective of the present invention is to provide a support for volatile substance-diffusing wicks which prevents, or at least limits, the accidental spillage of the liquid inside the container where the support is mounted.

Another objective of the present invention is to provide a support for volatile substance-diffusing wicks which prevents the wick from being removed for reuse.

### Description of the Invention

The mentioned drawbacks are solved with the support for wicks of the invention, having other advantages that will be described below.

The support for volatile substance-diffusing wicks according to the present invention is defined in claim 1 and it comprises a housing for placing a volatile substance-diffusing wick, and said housing is formed by a plurality of elastic extensions defining a funnel shape.

The wick can thereby be placed in said housing and the elastic extensions will hold said wick to prevent the removal thereof, preventing reuse. Furthermore, said funnel-shaped elastic extensions prevent the liquid from coming out accidentally, or limit same, when the support according to the present invention is mounted in a liquid-filled container.

Advantageously, the support for volatile substance-diffusing wicks according to the present invention also comprises at least one holding element for holding the support on a container, that is a plurality of legs, each with a protrusion at its distal end.

Said plurality of elastic extensions are spaced from one another by means of slots, and each elastic extension has a trapezoidal shape.

The support for volatile substance-diffusing wicks also preferably comprises a circumferential flange, particularly placed in the upper part thereof, according to its usage position.

Advantageously, the support for volatile substance-diffusing wicks is made of an elastic plastic material, such as polyethylene terephthalate (PET), polyethylene (PE), polypropylene (PP), acrylonitrile butadiene styrene (ABS), nylon, or any other molded material.

### Brief Description of the Drawings

To better understand what has been set forth, drawings in which a practical embodiment is schematically shown only by way of non-limiting example are attached.
Figure 1 is a side perspective view of the support for volatile substance-diffusing wicks according to the present invention;
Figure 2 is a top perspective view of the support for volatile substance-diffusing wicks according to the present invention; and
Figure 3 is an elevational sectioned view of the support for volatile substance-diffusing wicks according to the present invention, placed in a container.

### Description of a Preferred Embodiment

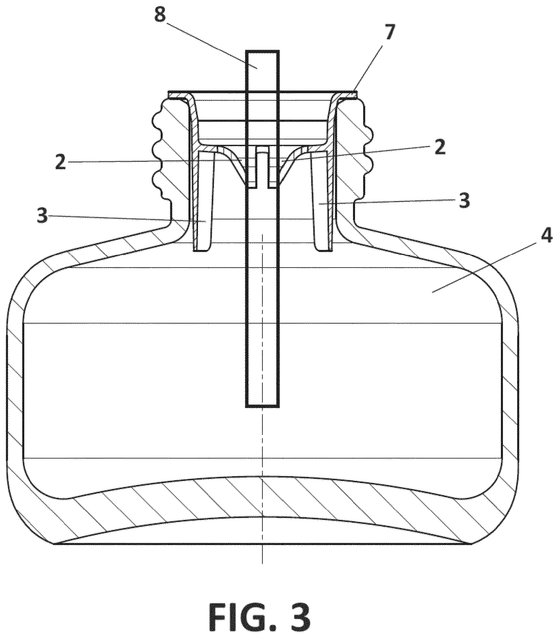

As shown in the drawings, the support for volatile substance-diffusing wicks according to the present invention is placed in a container 4, for example, a bottle, containing liquid which allows the diffusion of volatile substances, particularly perfume. Specifically, the support according to the present invention is coupled in the mouth of said container 4, as can be seen in Figure 3.

The wick support according to the present invention comprises a housing 1 for placing a wick 8, depicted in Figure 3. In this position, the wick 8 is placed substantially vertical in its usage position, being in contact with the liquid of the container 4 and is exposed to the open air to allow diffusion of the volatile substances.

The wick 8 is flexible and can be any soft material with capillary properties, such as textiles, cords made with different materials, non-woven fabrics, natural and/or artificial fibers, among others.

If desired, the wick 8 can be connected to a fragrance-diffusing element (not depicted in the drawings), for example, an element having a flower shape or other shapes, woods with capillary properties, or it can be a suitable cord wick acting as the fragrance diffuser itself.

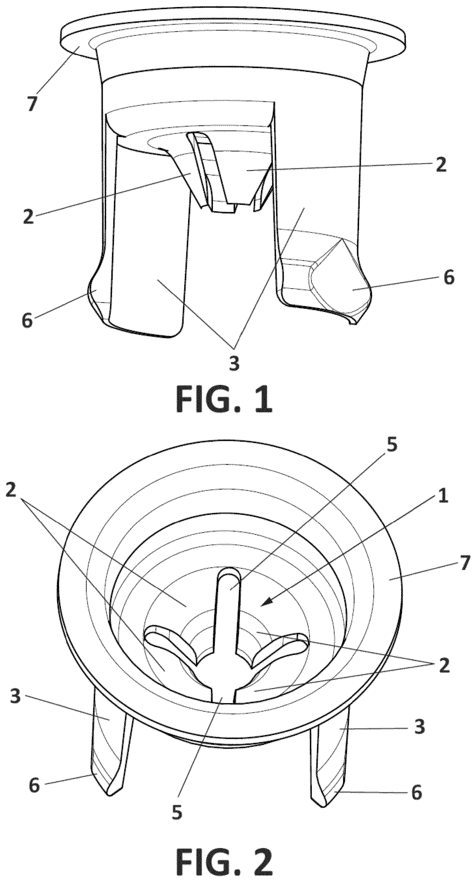

Said housing 1 is formed by a plurality of elastic extensions 2, defining a central hole between them, as seen in Figure 2. Said elastic extensions 2 as a whole define a funnel shape which prevents the wick 8 from being removed for reuse and the liquid inside the container 4 from coming out, for example, if said container 4 tips over, or at least limits same.

Said elastic extensions 2 are preferably spaced from one another by means of slots 5, and each elastic extension 2 has a substantially trapezoidal shape, as can be seen in Figures 1 and 2.

Therefore, by introducing the wick 8 in the housing 1, the elastic extensions 2 will elastically deform adapting to the width or diameter of the wick 8, creating a close contact between the wick 8 and the elastic extensions 2, with the wick 8 being held and a substantially liquid-tight closure being formed.

To hold the support in place in the container 4, the support comprises holding means formed, according to the depicted embodiment, by means of holding legs 3 comprising a protrusion 6 at their distal end, i.e., at the end that is introduced the farthest into the container 4, as seen in Figure 3.

These holding legs 3 must be elastic enough to allow the introduction thereof into the container 4 and also to hold the support to prevent its removal, preventing tampering of the support.

Furthermore, the support according to the present invention also comprises a circumferential flange 7 in its upper part, in the usage position depicted in Figure 3. This circumferential flange 7 abuts with the upper edge of the container 4, preventing the support from being completely introduced into the container 4.

The support is made entirely by means of molding, preferably of a plastic material with elastic features, such as polyethylene terephthalate (PET), polyethylene (PE), polypropylene (PP), acrylonitrile butadiene styrene (ABS), nylon, or any other molded material, although it may be any suitable material.

Furthermore, the elastic extensions 2 forming the housing 1 can be made from a suitable elastic material different from the rest of the support.

Although reference has been made to a specific embodiment of the invention, it is obvious for one skilled in the art that the support for wicks described is susceptible to a number of variations and modifications, and that all the mentioned details can be replaced with other technically equivalent details without departing from the scope of protection defined by the attached claims.

## Claims

1. Support for volatile substance-diffusing wicks, comprising a housing (1) for placing a volatile substance-diffusing wick, said housing (1) being formed by a plurality of elastic extensions (2), which extend inwardly to define a central hole and a funnel shape, wherein the elastic extensions are configured to elastically hold a wick placed in the central hole; wherein the support also comprises at least one holding element (3) for holding the support in a container,
**characterized in that** said at least one holding element (3) is a plurality of elastic legs, each with a radially outwardly protrusion (6) at its distal end, the distal end being the end that in use is introduce the farthest into the container,
wherein said plurality of elastic extensions (2) are spaced from one another by means of slots (5), and
wherein each elastic extension (2) has a trapezoidal shape.

2. Support for volatile substance-diffusing wicks according to claim 1, comprising a circumferential flange (7).

3. Support for volatile substance-diffusing wicks according to any of the preceding claims, which is made of an elastic plastic material.

## Patentansprüche

1. Halterung für Dochte zur Abgabe flüchtiger Substanzen, umfassend eine Aufnahme (1) zur Anordnung eines Dochtes zur Abgabe flüchtiger Substanzen, wobei die Aufnahme (1) durch eine Vielzahl elastischer Fortsätze (2) gebildet ist, die sich nach innen erstrecken, um eine zentrale Öffnung und eine Trichterform zu definieren, wobei die elastischen Fortsätze dazu ausgebildet sind, einen in der zentralen Öffnung angeordneten Docht elastisch zu halten; wobei die Halterung ferner mindestens ein Halteelement (3) zum Halten der Halterung in einem Behälter umfasst,
**dadurch gekennzeichnet, dass** das mindestens eine Halteelement (3) eine Vielzahl elastischer Schenkel ist, von denen jeder an seinem distalen Ende einen radial nach außen gerichteten Vorsprung (6) aufweist, wobei das distale Ende dasjenige Ende ist, das bei Gebrauch am weitesten in den Behälter eingeführt wird,
wobei die Vielzahl elastischer Fortsätze (2) durch Schlitze (5) voneinander beabstandet sind und
wobei jeder elastische Fortsatz (2) eine trapezförmige Gestalt aufweist.

2. Halterung für Dochte zur Abgabe flüchtiger Substanzen nach Anspruch 1, umfassend einen umlaufenden Flansch (7).

3. Halterung für Dochte zur Abgabe flüchtiger Substanzen nach einem der vorhergehenden Ansprüche, die aus einem elastischen Kunststoffmaterial hergestellt ist.

## Revendications

1. Support pour mèches de diffusion de substance volatile, comprenant un boîtier (1) pour placer une mèche de diffusion de substance volatile, ledit boîtier (1) étant formé par une pluralité d'extensions élastiques (2), qui s'étendent vers l'intérieur pour définir un trou central et une forme d'entonnoir, dans lequel les extensions élastiques sont configurées pour maintenir élastiquement une mèche placée dans le trou central ;
dans lequel le support comprend également au moins un élément de maintien (3) pour maintenir le support dans un récipient,
**caractérisé en ce que** ledit au moins un élément de maintien (3) est une pluralité de pattes élastiques, chacune avec une saillie radialement vers l'extérieur (6) à son extrémité distale, l'extrémité distale étant l'extrémité qui, en utilisation, est introduite le plus loin dans le récipient,
dans lequel ladite pluralité d'extensions élastiques (2) sont espacées les unes des autres au moyen de fentes (5), et
dans lequel chaque extension élastique (2) présente une forme trapézoïdale.

2. Support pour mèches de diffusion de substance volatile selon la revendication 1, comprenant une bride circonférentielle (7).

3. Support pour mèches de diffusion de substance volatile selon l'une quelconque des revendications précédentes, qui est constitué d'un matériau plastique élastique.
